(19) Europäisches Patentamt
European Patent Office
Office européen des brevets

(11) Veröffentlichungsnummer: **0 146 784**
**B1**

(12) **EUROPÄISCHE PATENTSCHRIFT**

(45) Veröffentlichungstag der Patentschrift: **08.03.89**

(21) Anmeldenummer: **84113999.1**

(22) Anmeldetag: **20.11.84**

(51) Int. Cl.[4]: **C 07 C 47/127**, C 07 C 47/198, C 07 C 49/175, C 07 C 49/185, C 07 C 45/40, C 07 C 43/303, C 07 C 41/48

(54) **Verfahren zur Herstellung von Glyoxal, Alkylglyoxalen und von deren Acetalen.**

(30) Priorität: **21.12.83 DE 3346266**

(43) Veröffentlichungstag der Anmeldung: **03.07.85 Patentblatt 85/27**

(45) Bekanntmachung des Hinweises auf die Patenterteilung: **08.03.89 Patentblatt 89/10**

(84) Benannte Vertragsstaaten: **AT BE CH DE FR GB IT LI LU NL SE**

(56) Entgegenhaltungen:
**DE-A-2 514 001**

**CHEMICAL ABSTRACTS, Band 53, Nr. 7, 10. April 1959, Spalte 6090 c-d, Columbus, Ohio, US**

(73) Patentinhaber: **Chemie Linz Gesellschaft m.b.H., St.Peter- Strasse 25, A-4021 Linz (AT)**
(84) Benannte Vertragsstaaten: **BE CH FR GB IT LI LU NL SE AT**

(73) Patentinhaber: **Lentia Gesellschaft mit beschränkter Haftung, Arabellastrasse 4 Postfach 81 05 08, D-8000 München 81 (DE)**
(84) Benannte Vertragsstaaten: **DE**

(72) Erfinder: **Sajtos, Alexander, Dipl.- Ing., Hartackerstrasse 48, A-4020 Linz (AT)**
Erfinder: **Wechsberg, Manfred, Dipl.- Ing. Dr., Im Blumengrund 0/35, A-4020 Linz (AT)**
Erfinder: **Roithner, Erich, Stülzgasse 14, A-4020 Linz (AT)**

**EP 0 146 784 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von Glyoxal, Alkylglyoxalen und von deren Acetalen aus α,β-ungesättigten Dialkylacetalen.

Verfahren zur Herstellung von Monoacetalen des Glyoxals aus Dialkylacetalen von α,β-ungesättigten Aldehyden mittels eines Ozonolyse- und Reduktionsprozesses sind vereinzelt beschrieben. In den Chemischen Berichten 36 (1903), Seite 1935, ist angegeben, daß bei der Einwirkung von Ozon auf Acroleindiäthylacetal in wässeriger Emulsion das Diäthylmonoacetal des Glyoxals entsteht. Es werden dort jedoch keine Ausbeuten angegeben und die erhaltene Substanz wurde nicht näher charakterisiert. Die in der DE-OS-2 514 001 angegebene Nacharbeitung des in den Chem. Berichten beschriebenen Verfahrens führte aber zur Erkenntnis, daß das bei der Ozonisierung entstehende Reaktionsgemisch ohne erkennbare Ursache brisant explodieren kann. Die Untauglichkeit des bekannten Verfahrens wird aus Beispiel 4 der Offenlegungsschrift deutlich, wo angegeben ist, daß die durch Einleiten eines $O_2/O_3$ Gemisches in eine wässerige Lösung von Acroleindimethylacetal entstandene Reaktionsmischung unter völliger Zerstörung der Apparatur brisant explodierte.

Zur Vermeidung dieser Schwierigkeiten wird in der DE-OS-2 514 001 daher ein anderes Verfahren zur Herstellung von Glyoxalmonoacetalen vorgeschlagen, bei dem nicht Acetale des Acroleins, sondern die schwerer zugänglichen und teureren Acetale des Crotonaldehyds als Ausgangsstoffe in organischen Lösungsmitteln mit Ozon umgesetzt und die Ozonisierungsprodukte anschließend bevorzugt durch katalytische Hydrierung reduktiv aufgespalten werden. Bei der Durchführung dieses Verfahrens wird in eine Lösung des Crotonaldehydacetals so lange Ozon im Überschuß eingeleitet, bis es in merklicher Menge das Reaktionsgemisch wieder verläßt. Das überschüssige Ozon muß vor der reduktiven Aufspaltung des Ozonisierungsprodukts zum Schutz des Hydrierkatalysators vor Aktivitätsverlusten durch Spülung der Reaktionslösung mit einem Inertgas, beispielsweise mit Stickstoff, in einem weiteren Arbeitsgang wieder entfernt werden. Zur Durchführung der Hydrierung setzt man dann dem bei der Ozonolyse erhaltenen Reaktionsgemisch pro 100 ml 1 bis 3 g des Katalysators, der bevorzugt ein Edelmetallkatalysator ist, direkt zu und leitet bis zur Sättigung Wasserstoff ein. Die Druckschrift gibt keine Auskunft über Möglichkeiten zur Regenerierung oder Wiederverwendung der eingesetzten Edelmetallkatalysatoren nach Beendigung der Hydrierung.

Bei Einsatz von Acetalen des Crotonaldehyds bleibt das Verfahren auf die Herstellung von Glyoxalmonoacetalen beschränkt. Acetale von Alkylglyoxalen sind nicht zugänglich und in der DE-OS-2 514 001 auch nicht beschrieben.

Die dem bekannten Verfahren anhaltenden Nachteile können gemäß der vorliegenden Erfindung überraschenderweise durch ein einfaches und ökonomisches Verfahren vermieden werden, bei dem man ein Dialkylacetal des Acroleins oder eines Alkylacroleins unter Vermeidung jeglichen Überschusses mit einem Moläquivalent Ozon umsetzt und das peroxidhältige Ozonisierungsprodukt in verdünnter Lösung bei einer sehr niedrigen Konzentration an Peroxiden durch katalytische Hydrierung rasch reduziert.

Gegenstand der vorliegenden Erfindung ist demnach ein Verfahren zur Herstellung von Glyoxal, Alkylglyoxalen und von deren Acetalen der allgemeinen Formel

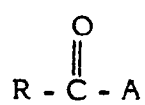

I,

worin

R für Wasserstoff oder einen geradkettigen oder verzweigten $C_1$ bis $C_6$-Alkylrest und

A für den Rest -CH = O oder 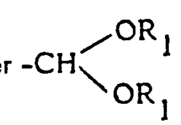

steht,

wobei $R_1$ einen geradkettigen oder verzweigten $C_1$- bis $C_6$-Alkylrest darstellt,

durch Ozonolyse eines α,β-ungesättigten Dialkylacetals gefolgt von katalytischer Hydrierung des Ozonisierungsprodukts, wobei das Verfahren dadurch gekennzeichnet ist, daß man

a) ein Dialkylacetal des Acroleins oder eines α-Alkylacroleins der allgemeinen Formel

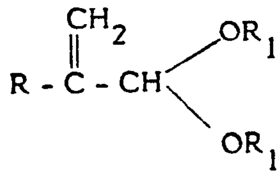

II,

in der R und $R_1$ die in Formel I angegebene Bedeutung haben, in einem organischen Solvens löst und bei Temperaturen von -80 bis 0°C mit der äquivalenten Menge Ozon umsetzt,

b) die bei der Ozonisierung erhaltene Lösung anschließend in eine Suspension des Hydrierkatalysators in dem in Stufe a) verwendeten Lösungsmittel kontinuierlich in einer solchen Dosierung einspeist, daß über den ganzen Verlauf der Hydrierung in der Hydrierlösung ein Peroxidgehalt von maximal 0,1 Mol/l eingestellt und/oder aufrecht erhalten wird, und die Ozonisierungsprodukte bei einem pH-Wert von 2 bis 7 und Temperaturen von 15 bis 45°C durch Einleiten von Wasserstoff unter einem Druck von 1 bis 20 bar reduktiv aufspaltet, worauf man

c) gewünschtenfalls die entstandenen Acetale der Formel I mit der Bedeutung für

A ist 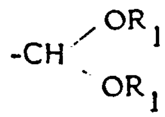

durch Erhitzen mit Wasser in Anwesenheit von Säuren oder Basen zum entsprechenden Glyoxal der Formel I mit der Bedeutung für

A ist -CH=O

hydrolytisch spaltet.

In bevorzugter Weise werden solche Alkylacroleindialkylacetale der Formel II umgesetzt, in der R und $R_1$ unabhängig voneinander für einen geradkettigen oder verzweigten $C_1$- bis $C_4$-Alkylrest stehen. Besonders bevorzugt umgesetzt werden solche Acetale der Formel II, in der R und $R_1$ unabhängig voneinander Methyl oder Äthyl bedeuten, wobei die Bedeutung Methyl für R und $R_1$ wiederum ganz besonders bevorzugt ist.

Die Ozonisierung wird vorzugsweise bei Temperaturen von -30 bis 0°C durchgeführt; wobei die Einhaltung einer Temperatur von -15 bis -5°C wiederum besonders bevorzugt ist. Beim erfindungsgemäßen Verfahren wird das jeweils zur Umsetzung gelangende Acroleindialkylacetal mit genau der äquivalenten Menge an Ozon behandelt, wobei unter den angegebenen Verfahrensbedingungen Ozon quantitativ umgesetzt wird und stöchiometrische Mengen des Acetals der Formel II verbraucht werden. Durch die Vermeidung eines Ozonüberschusses kann die bei der Ozonisierung von Acetalen beobachtete Neigung zum spontanen, explosionsartigen Zerfall verhindert werden und es muß nach Beendigung der Ozonisierung auch nicht mehr dafür Sorge getragen werden, daß überschüssiges oder nicht umgesetztes Ozon vor der Hydrierung aus dem Reaktionsgemisch vertrieben wird.

Die Umsetzung des Acroleinacetals mit Ozon in Stufe a) erfolgt in einem organischen Lösungsmittel. Als organische Lösungsmittel kommen reine oder chlorierte Kohlenwasserstoffe wie z. B. Cyclohexan oder Petroläther, Gemische von Kohlenwasserstoffen, Tetrachlorkohlenstoff, Chloroform, Methylenchlorid, Essigester oder vorteilhafterweise niedere aliphatische Alkohole in Frage. Bevorzugte Lösungsmittel sind vor allem Methanol oder Äthanol wobei wiederum die Verwendung von Methanol besonders bevorzugt ist.

Die katalytische Hydrierung der Ozonolyseprodukte wird beim erfindungsgemäßen Verfahren in stark verdünnter Lösung durchgeführt, wobei durch geeignete Maßnahmen und Vorrichtungen dafür Sorge getragen wird, daß während der gesamten Hydrierung in der Hydrierlösung ein Peroxidgehalt von höchstens 0,1 Mol/l, vorzugsweise von höchstens 0,05 Mol/l und insbesondere von höchstens 0,02 Mol/l eingestellt und aufrecht erhalten wird. Zur praktischen Durchführung wird beispielsweise in einem Hydrierreaktor eine Suspension des Katalysators in dem in Stufe a) bei der Ozonisierung verwendeten Lösungsmittel, bevorzugt in einem niedrigen aliphatischen Alkohol, ganz bevorzugt in Methanol, vorgelegt und die bei der Ozonisierung erhaltene Lösungsmittels einer regelbaren Dosiervorrichtung kontinuierlich eingespeist. Bei der Zugabe der Ozonolyselösung zu Beginn und im Verlauf der Hydrierung ist selbstverständlich darauf zu achten, daß durch die zugeführte Menge der peroxidhaltigen Ozonisierungsprodukte der oben angegebene Peroxidgehalt in der Hydrierlösung nicht überschritten wird.

Durch die geringe Konzentration an peroxidhältigen Ozonisierungsprodukten während des eigentlichen Hydrierungsvorganges ist das Mengenverhältnis von Katalysator und zu reduzierendem Substrat sehr günstig, sodaß auch bei sparsamen Einsatz des Katalysators eine rasche Reduktion gewährleistet ist. Auf diese Weise wird auch die bei hohen Peroxidkonzentrationen sonst zu beobachtende Vergiftung und der damit verbundene Aktivitätsverlust des Katalysators verhindert.

Im gesamten gesehen kann aber durch die kontinuierliche Einspeisung eine große Menge an Ozonisierungsprodukt in einem verhältnismäßig kleinen Volumen reduziert werden, wodurch in der Endstufe

des Verfahrens konzentrierte Lösungen der Acetale des Glyoxals oder Alkylglyoxals anfallen und neben Lösungsmittel selbst auch Zeit und Kosten bei der destillativen Entfernung der Lösungsmittei gespart werden.

Als Katalysatoren eignen sich die für Hydrierungen üblicherweise verwendeten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet, insbesondere Platinkatalysatoren ohne Trägermaterial. Bei Pulverkontakten eignen sich als Trägermaterial beispielsweise Kohle, Aluminium, Silikagel oder Kieselgur. Die Ausbeuten sind beim erfindungsgemäßen Verfahren an sich unabhängig von der eingesetzten Katalysatormenge, jedoch empfiehlt es sich zur Erzielung einer ausreichenden Hydriergeschwindigkeit die genannten Katalysatoren in Edelmetallmengen von 0,1 bis 5 Gew. %, vorzugsweise von 0,5 bis 2 Gew.%, bezogen auf die jeweils pro Stunde eingespeiste Gesamtmenge an ozonisiertem Acrolein- oder Alkylacroleindialkylacetal, vorzulegen.

Nach Beendigung des Hydriervorgangs wird der Katalysator aus dem Reaktionsgemisch abgetrennt und ohne Regenerierung für die reduktive Aufspaltung in weiteren Reaktionszyklen verwendet, wobei kein Aktivitätsverlust des Katalysators beobachtet wird.

Die Hydrierung wird so lange fortgesetzt bis keine Wasserstoff-Aufnahme mehr feststellbar ist. Beim erfindungsgemäßen Verfahren werden zur Reduktion der Ozonisierungsprodukte äquivalente Mengen an Wasserstoff verbraucht. Die Menge an Wasserstoff, die bei der Hydrierung verwendet werden kann, reicht von einem Moläquivalent bis zu einem mehrfachen molaren Überschuß. Die Verwendung von überschüssigem Wasserstoff bringt an sich keine Vorteile und ist nur zweckmäßig, um eine ausreichende Versorgung des Hydriergemisches mit Wasserstoff sicherzustellen.

Die Hydrierung erfolgt beim erfindungsgemäßen Verfahren in vorteilhafterweise unter praktisch drucklosen Bedingungen. Unter praktisch drucklosen Bedingungen sollen hier Drücke von 1 bis etwa 3 bar verstanden werden, wie das in der Technik üblich ist, um das Eindringen von Luft in den Hydrierreaktor zu verhindern. Auf diese Weise ist die Reduktion der Ozonisierungsprodukte technisch sehr einfach durchzuführen. Es ist aber auch möglich, die Hydrierung bei einem Druck bis zu 20 bar durchzuführen und dadurch die Hydrierungsgeschwindigkeit zu steigern.

Die Reduktion verläuft exotherm und wird gemäß einer bevorzugten Ausführungsform der vorliegenden Erfindung bei 20 bis 40° C, insbesondere bei Temperaturen im Bereich von 35 bis 40° C durchgeführt.

Vorteilhafterweise wird während der Hydrierung ein pH-Wert von 2 bis 5 eingehalten. Da sich im Verlaufe der Hydrierung in geringen Mengen saure Nebenprodukte bilden, ist zur Einhaltung des gewünschten pH-Wertes die dosierte Zugabe einer Base, vorteilhafterweise von verdünnter Natronlauge notwendig.

Zweckmäßigerweise werden nach Beendigung der Hydrierung die im Reaktonsgemisch enthaltenen Kationen der jeweils eingesetzten Base vor dem Abdestillieren des Lösungsmittels und der Isolierung der Acetale der Formel I aus dem Reaktionsgemisch wieder entfernt, indem die Reaktionslösung beispielsweise mit einem sauren Ionenaustauscher behandelt wird. Es können zu diesem Zweck handelsübliche Ionenaustauscher in der H-Form verwendet werden, beispielsweise Polystyrolharze, die Sulfonsäurereste tragen. Zur Aufarbeitung werden Lösungsmittel, Reaktionswasser und eventuell vorhandene flüchtige Begleitprodukte vorteilhafterweise bei vermindertem Druck abdestilliert und die Acetale der Formel I mit der Bedeutung A ist

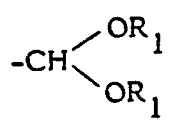

bevorzugt durch Rektifizieren mit oder ohne Zugabe von Hilfsmitteln in reinem Zustand gewonnen. Im erfindungsgemäßen Verfahren werden die Acetale auf diese Weise in einer Reinheit von mehr als 98 % und überraschenderweise frei von Nebenprodukten erhalten.

Die erfindungsgemäß erhaltenen Acetale können in einfacher Weise zu Glyoxal oder den entsprechenden Alkylglyoxalen der Formel I der Bedeutung A ist -CH=O hydrolytisch aufgespalten werden, beispielsweise durch Erhitzen in Wasser unter Zugabe von katalytischen Mengen an Säuren. Besonders vorteilhaft ist es die hydrolytische Spaltung der Acetale in Anwesenheit eines stark sauren Ionenaustauschers als Katalysator durchzuführen, da mit dieser Methode der Katalysator nach Beendigung der Hydrolyse auf einfache Weise wieder aus dem Reaktionsgemisch abgetrennt werden kann. Dabei ist es aber nicht notwendig, die Acetale nach der Hydrierung zu isolieren, sondern die Hydrolyse kann nach beendeter Hydrierung und Abtrennung des Katalysators direkt in der Hydrierlösung vorgenommen werden.

Die als Ausgangssubstanzen benötigten α,β-ungesättigten Acetale der Formel II können in bekannter Weise beispielsweise durch Acetalisierung von α,β-ungesättigten Aldehyden unter Zusatz wasserentziehender Mittel nach dem in Org. Synth. IV, Seiten 21-22 beschriebenen Verfahren oder auch durch Chlorierung gesättigter Aldehyde, Acetalisierung und HCl-Abspaltung, beschrieben im Chemischen Zentralblatt, 1937: 1, Seiten 5098 ff., hergestellt werden.

Die Verfahrensprodukte der Formel I sind wertvolle Ausgangs- und Zwischenprodukte, aus denen man beispielsweise Substanzen mit großer biologischer und pharmakologischer Bedeutung herstellen kann.

Das erfindungsgemäße Verfahren wird in den folgenden Beispielen näher erläutert.

**Beispiel 1:** Glyoxaldimethylacetal

918 g (9 mol) Acroleindimethylacetal, gelöst in 6 l Methanol werden bei -15 bis -10°C durch Einleiten eines Stromes von 1000 l Sauerstoff je Stunde, der 4 Gew.-% (= 1.17 Mol/Stunde) Ozon enthält, mit der äquivalenten Menge Ozon umgesetzt. Dabei wird Ozon quantitativ aufgenommen und der Restgehalt an Acroleindimethylacetal beträgt nach beendeter Ozonisierung weniger als 1 % der Ausgangskonzentration.

Die bei der Ozonisierung erhaltene Lösung wird portioniert und über ein Dosiergefäß in einen Hydrierreaktor, in dem eine Suspension von 5 g Platin hergestellt durch Reduktion von $PtO_2$ mit $H_2$ in situ, in 1 l Methanol vorgelegt wird und der mit Wasserstoff gefüllt ist, kontinuierlich in solchen Mengen eingespeist, daß der Peroxidgehalt in der Hydrierlösung zu Beginn und im Verlaufe der gesamten Hydrierung maximal 0.02 Mol/l beträgt. Unter kräftigem Rühren und Wasserstoffzugabe wird bis zur negativen Peroxidprobe hydriert, wobei die Temperatur durch Kühlung von außen auf 35 bis 40°C gehalten wird. Der verbrauchte Wasserstoff wird laufend aus einem Vorratsgefäß ergänzt und durch Zugabe von methanolischer NaOH wird in der Lösung ein pH-Wert von 2 bis 4 eingehalten. Insgesamt werden während der Hydrierung 159 Normalliter $H_2$ (79 % d. Theorie) aufgenommen.

Zur Aufarbeitung wird der Inhalt des Hydrierreaktors bis auf einen Rückstand von 1 l über eine Fritte abgesaugt. Die aus dem Hydrierreaktor abgesaugte Lösung wird mit einem sauren Ionenaustauscher (Lewatit) behandelt und das Lösungsmittel sowie flüchtige Begleitprodukte werden unter vermindertem Druck abdestilliert. Der das Reaktionsprodukt enthaltende Rückstand wird durch Rektifikation gereinigt und man erhält 758 g (7,29 mol) Glyoxaldimethylacetal, entsprechend einer Ausbeute von 81 % der Theorie.

Der im Hydrierreaktor im kleineren Teil der Hydrierlösung zurückgebliebene Katalysator wird ohne Regenerierung oder Aufarbeitung für die reduktive Spaltung wiederverwendet, indem von neuem ozonisierte Lösung von Acroleindimethylacetal über das Dosiergefäß in den Reaktor eingespeist und der Hydriervorgang unter den oben angegebenen Reaktionsbedingungen wiederholt wird.

**Beispiel 2:** Methylglyoxaldimethylacetal

In einem Reaktor werden 1044 g (9 mol) Methacroleindimethylacetal, gelöst in 6 l Methanol vorgelegt und durch Einleiten eines $O_2/O_3$ Gemisches (1000 l $O_2$/h, 56 g $O_3$/h) bei Temperaturen von -10 bis -5°C wie in Beispiel 1 angegeben mit Ozon umgesetzt. Dabei wird Ozon quantitativ aufgenommen und eine stöchiometrische Menge an Methacroleindimethylacetal verbraucht. Der Restgehalt an Methacroleindimethylacetal beträgt nach beendeter Ozonisierung weniger als 0,8 % der Ausgangskonzentration.

Die bei der Ozonisierung erhaltene Lösung wird über ein Dosiergefäß in einen Hydrierreaktor, in dem eine Suspension von 4 g Pt in 1 l Methanol vorgelegt wird, unter Rühren und Wasserstoff-Einleitung in solchen Dosen eingespeist, daß der Peroxidgehalt im Hydrierreaktor zu Beginn und im Verlaufe der Hydrierung 0.05 Mol/l nicht übersteigt. Das Reaktionsgemisch wird durch Kühlung von außen auf einer Temperatur von 25 bis 30°C gehalten und über eine automatische pH-Kontrolle wird durch Zudosierung von methanolischer NaOH ein pH-Wert von 4 - 5 eingestellt. Nach Beendigung der Zugabe der Ozonisierungslösung wird das Reaktionsgemisch innerhalb von 5 bis 10 Minuten peroxidfrei. Die $H_2$-Aufnahme beträgt 180 N l (89,3 % der Theorie).

Zur Aufarbeitung wird der Inhalt des Hydrierreaktors über eine Fritte abgesaugt und die Reaktionslösung mit einem sauren Ionenaustauscher (Lewatit) natriumfrei gemacht. Methanol und die bei der reduktiven Spaltung entstehenden flüchtigen Begleitprodukte werden am Dünnschichtverdampfer abdestilliert und der das Reaktionsprodukt enthaltende Rückstand wird mit Natronlauge neutralisiert. Das aus der Hydrierung stammende Wasser wird dann azeotrop mit Petroläther ausgekreist und der das Reaktionsprodukt enthaltende Rückstand unter Zugabe von Harnstoff rektifiziert.

Man erhält 956 g (8,1 mol) Methylglyoxaldimethylacetal, entsprechend einer Ausbeute von 90 % der Theorie, mit einem $K_{p100}$ = 76°C.

**Beispiel 3:**

Der in Beispiel 2 beschriebene Ozonolyse- und Reduktionsprozess mit Methacroleindimethylacetal wird wiederholt und der Inhalt des Hydrierreaktors nach beendeter Hydrierung so weit abgesaugt, daß 1 l der Lösung und der Katalysator im Hydrierreaktor zurückbleiben. In diesen Rückstand wird eine neue Charge Ozonisierungsprodukt unter den oben angegebenen Bedingungen eindosiert und unter Wasserstoffzufuhr reduktiv gespalten. Bei insgesamt 10 aufeinanderfolgenden Reduktionszyklen bleibt der Wasserstoff verbrauch mit einem Gesamtverbrauch vn 1774 Normallitern (79,2 mol $H_2$, 88 % der Theorie) in jedem Reaktionszyklus ungefähr gleich wie im ersten Ansatz. Die Gesamtausbeute an Methylglyoxaldimethylacetal beträgt 9511 g (80,5 mol), entsprechend 89,4 % der Theorie.

**Beispiel 4:** Methylglyoxaldiäthylacetal

1 l einer äthanolischen Lösung von 216 g (1,5 mol) Methacroleindiäthylacetal werden analog zu der in Beispiel 1 angegebenen Arbeitsweise mit Ozon ungesetzt und anschließend hydriert. Die $H_2$-Aufnahme beträgt 30 Normalliter (89,3 % der Theorie.)

Nach der Aufarbeitung, die durch Behandeln des Reaktionsgemisches mit einem sauren Ionenaustauscher, Abdestillieren des Lösungsmittels und Rektifizieren des Reakionsproduktes analog zu den Angaben in Beispiel 2 erfolgt, erhält man 189 g reines Methylglyoxaldiäthylacetal vom $K_{p25}$ = 69° C, entsprechend einer Ausbeute von 86.3 % der Theorie.

**Beispiel 5:** Methylglyoxaldi-n-butylacetal

1 l einer Lösung von 300 g (1,5 mol) Methacroleindi-n-butylacetal in Äthanol werden analog zu der in Beispiel I angegebenen Arbeitsweise mit Ozon umgesetzt und anschließend hydriert. Die $H_2$-Aufnahme beträgt 28,5 Normalliter (84,8 % der Theorie.)

Nach der Aufarbeitung, die durch Behandeln der Reaktionslösung mit einem sauren Ionenaustauscher (Lewatit), Abdestillieren des Lösungsmittels und Rektifizieren des Reaktionsproduktes analog zu den Angaben in Beispiel 2 erfolgt, erhält man 245 g reines Methylglyoxaldi-n-butylacetal vom $K_{p15}$ = 104° C, entsprechend einer Ausbeute von 81 % der Theorie.

**Beispiel 6:** Äthylglyoxaldimethylacetal

1 l einer Lösung von 195 g (1,5 mol) Äthylacroleindimethylacetal in Methanol werden analog zu der in Beispiel 1 angegebenen Arbeitsweise bei -25° C bis -30° C mit Ozon umgesetzt und anschließend hydriert. Die $H_2$-Aufnahme beträgt 29,55 Normalliter (87,8 % der Theorie). Nach der Aufarbeitung, die durch Abtrennen des Katalysators, Behandeln der Reaktionslösung mit einem sauren Ionenaustauscher, Abdestillieren des Lösungsmitteis und Rektifizieren des Reaktionsproduktes analog zu den Angaben in Beispiel 2 erfolgt, erhält man 169 g Äthylglyoxaldimethylacetal vom $K_{p15}$ = 52° C, entsprechend einer Ausbeute von 85,4 % der Theorie.

**Beispiel 7:** n-Butylglyoxaldimethylacetal

1 l einer Lösung von 237 g (1,5 mol) n-Butylacroleindimethylacetal in Methanol werden analog zu der in Beispiel 1 angegebenen Arbeitsweise mit Ozon umgesetzt und anschließend hydriert. Die $H_2$-Aufnahme beträgt 29 Normalliter.

Nach der Aufarbeitung, die durch Abtrennen des Katalysators, Behandeln der Reaktionslösung mit einem sauren Ionenaustauscher, Abdestillieren des Lösungsmittels und Rektifizieren des Reaktionsprodukts analog zu den Angaben in Beispiel 2 erfolgt, erhält man 199 g n-Butylglyoxaldimethylacetal vom $K_{p12}$ = 80° C, entsprechend einer Ausbeute von 82,9 % der Theorie.

**Beispiel 8:** Methylglyoxaldimethylacetal

1 l einer Lösung von 174 g (1,5 mol) Methacroleindimethylacetal in Äthylacetat werden bei -45 bis -50° C analog zu der in Beispiel 1 angegebenen Arbeitsweise mit der äquivalenten Menge Ozon umgesetzt. Zur Hydrierung wird eine Suspension von 5 g eines Katalysators, der 10 % Pd auf Aktivkohle enthält, in Äthylacetat in einem mit $H_2$ gefüllten Hydrierreaktor vorgelegt, und die bei der Ozonisierung erhaltene Lösung über ein Dosiergefäß in solchen Mengen eingespeist, daß der Peroxidgehalt in der Hydrierlösung zu Beginn und im Verlaufe der gesamten Hydrierung maximal 0,1 Mol/l beträgt und bis zur negativen Peroxidprobe bei 25 bis 30° C und einem pH-Wert von 3 bis 4 weiterhydriert.

Nach der Aufarbeitung, die durch Abtrennen des Katalysators, Behandeln der Reaktionslösung mit einem sauren Ionenaustauscher, Abdestillieren des Lösungsmittels und Rektifizieren des Reaktionsprodukts analog zu den Angaben in Beispiel 2 erfolgt, erhält man 108 g Methylglyoxaldimethylacetal vom $K_{p100}$ = 76° C, entsprechend einer Ausbeute von 61 % der Theorie.

**Beispiel 9:** Isobutylglyoxaldiäthylacetal

1 Liter einer Lösung von 242 g (1,3 mol) Isobutylacroleindiäthylacetal in Äthanol werden analog zu der in Beispiel 1 angegebenen Arbeitsweise mit Ozon umgesetzt und anschließend unter Einhaltung eines pH-Wertes von 4 bis 5 hydriert. Die $H_2$-Aufnahme beträgt 25,5 Normalliter (87,5 % der Theorie).

Nach der Aufarbeitung, die analog zu den Angaben in Beispiel 2 erfolgt, erhält man 205 g reines Isobutylglyoxaldiäthylacetal vom $K_{p25}$ = 88° C, entsprechend einer Ausbeute von 84 % der Theorie.

**Beispiel 10:** Hydrolytische Spaltung von Methylglyoxaldimethylacetal zu Methylglyoxal

118 g (1 mol) Methylglyoxaldimethylacetal und 250 g Wasser werden mit 5 g eines stark sauren Ionenaustauschers (Lewatit in der H+-Form) erhitzt und das Methanol-Wassergemisch abdestilliert. Man erhält so 198 g einer wässerigen Lösung von Methylglyoxal mit einem Gehalt von 35,9 Gew.-%. Dabei wird Methylglyoxaldimethylacetal in quantitativer Ausbeute zu Methylglyoxal hydrolytisch gespalten.

**Patentansprüche**

1. Verfahren zur Herstellung von Glyoxal, Alkylglyoxalen und von deren Acetaten der allgemeinen Formel

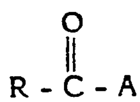 I,

worin

R für Wasserstoff oder einen geradkettigen oder verzweigten $C_1$- bis $C_6$-Alkylrest und

A für den Rest -CH=O oder 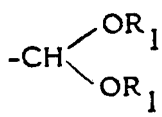

steht, wobei

$R_1$ einen geradkettigen oder verzweigten $C_1$- bis $C_6$-Alkylrest darstellt,

durch Ozonolyse eines α,β-ungesättigten Dialkylacetals gefolgt von katalytischer Hydrierung des Ozonisierungsprodukts, dadurch gekennzeichnet, daß man

a) ein Dialkylacetal des Acroleins oder eines α-Alkylacroleins der allgemeinen Formel

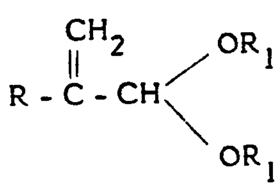 II,

in der R und $R_1$ die in Formel I angegebene Bedeutung haben, in einem organischen Solvens löst und bei Temperaturen von -80 bis 0° C mit der äquivalenten Menge Ozon umsetzt,

b) die bei der Ozonisierung erhaltene Lösung anschließend in eine Suspension des Hydrierkatalysators in dem in Stufe a) verwendeten Lösungsmittel kontinuierlich in einer solchen Dosierung einspeist, daß über den ganzen Verlauf der Hydrierung in der Hydrierlösung ein Peroxidgehalt von maximal 0,1 Mol/l eingestellt beziehungsweise aufrecht erhalten wird, und die Ozonisierungsprodukte bei einem pH-Wert von 2 bis 7 und Temperaturen von 15 bis 45° C durch Einleiten von Wasserstoff unter einem Druck von 1 bis 20 bar reduktiv aufspaltet, worauf man

c) gewünschtenfails die entstandenen Acetale der Formel I mit der Bedeutung für

A ist 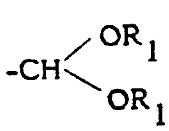

durch Erhitzen mit Wasser in Anwesenheit von Säuren oder Basen zum entsprechenden Glyoxal der Formel I

mit der Bedeutung für A ist -CH=O hydrolytisch spaltet.

2. Verfahren nach Anspruch 1, dadurch gekennzeichnet, daß man die Ozonisierung in Stufe a) bei Temperaturen im Bereich von -15 bis -5° C durchführt.

3. Verfahren nach den Ansprüchen 1 und 2 dadurch gekennzeichnet, daß man bei der Ozonisierung in Stufe a) und der reduktiven Aufspaltung der Ozonisierungsprodukte in Stufe b) einen niederen aliphatischen Alkohol als Lösungsmittel verwendet.

4. Verfahren nach Anspruch 3, dadurch gekennzeichnet, daß man bei der Ozonisierung in Stufe a) und der reduktiven Aufspaltung der Ozonisierungsprodukte in Stufe b) Methanol als Lösungsmittel verwendet.

5. Verfahren nach den Ansprüchen 1 bis 4, dadurch gekennzeichnet, daß zur reduktiven Aufspaltung der Ozonisierungsprodukte in Stufe b) in der Hydrierlösung ein Peroxidgehalt von höchstens 0,02 Mol/l eingestellt und/oder aufrecht erhalten wird.

6. Verfahren nach den Ansprüchen 1 bis 5, dadurch gekennzeichnet, daß zur reduktiven Spaltung in Stufe b) Platin ohne Trägermaterial als Katalysator verwendet wird.

7. Verfahren nach den Ansprüchen 1 bis 6, dadurch gekennzeichnet, daß die reduktive Spaltung in Stufe b) im Temperaturbereich von 35 bis 40° C vorgenommen wird.

8. Verfahren nach den Ansprüchen 1 bis 7, dadurch gekennzeichnet, daß während der reduktiven Spaltung in Stufe b) ein pH-Wert von 2 bis 5 eingestellt wird.

9. Verfahren nach den Ansprüchen 1 bis 8, dadurch gekennzeichnet, daß man die Acetale in Stufe c) durch Erhitzen mit Wasser in Anwesenheit eines stark sauren Ionenaustauschers spaltet.

10. Verfahren nach den Ansprüchen 1 bis 9, dadurch gekennzeichnet daß zur Umsetzung solche Acetale der Formel II eingesetzt werden, in der R und $R_1$ Methyl bedeuten.

## Claims

1. Process for the preparation of glyoxal, alkylglyoxals and acetals thereof of the general formula

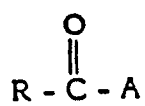 I,

wherein
R represents hydrogen or a linear or branched $C_1$ to $C_6$ alkyl radical and
A represents the radical -CH=O or

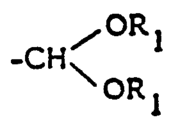

wherein $R_1$ represents a linear or branched $C_1$ to $C_6$ alkyl radical, by ozonolysis of an α,β-unsaturated dialkylacetal followed by catalytic hydrogenation of the ozonization product, characterized in that

a) a dialkylacetat of acrolein or of an α-alkylacrolein of the general formula

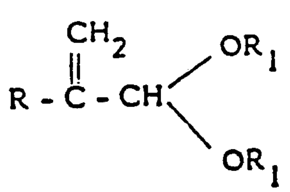 II,

in which R and $R_1$ have the meaning indicated in formula I, is dissolved in an organic solvent and reacted with the equivalent amount of ozone at temperatures from -80 to 0° C,

b) the solution obtained in the ozonization is then hydrogenated by being fed continuously into a suspension of a hydrogenation catalyst in the solvent used in stage a), at such a rate of metered addition that a peroxide content of not more than 0.1 mole/litre is set up and/or maintained in the hydrogenation solution during the entire course of the hydrogenation, and the ozonization products are cleaved reductively at a pH value of 2 to 7 and at temperatures from 15 to 45° C by passing in hydrogen under a pressure of 1 to 20 bar, after which,

c) if desired, the resulting acetals of the formula I in which A denotes

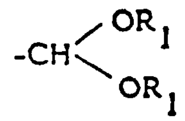

are cleaved hydrolytically by heating with water in the presence of acids or bases to give the corresponding glyoxal of the formula I in which A denotes -CH=O.

2. Process according to Claim 1, characterized in that the ozonization in stage a) is carried out at temperatures within the range from -15 to -5° C.

3. Process according to Claims 1 and 2, characterized in that a lover aliphatic alcohol is used as the solvent in the ozonization in stage a) and in the reductive cleavage of the ozonization products in stage b).

4. Process according to Claim 3, characterized in that methanol is used as the solvent in the ozonization in stage a) and in the reductive cleavage of the ozonization products in stage b).

5. Process according to Claims 1 to 4, characterized in that a peroxide content of not more than 0.02 mole/litre is set up and/or maintained in the hydrogenation solution for the reductive cleavage of the ozonization products in stage b).

6. Process according to Claims 1 to 5, characterized in that platinum without a support is used as the catalyst for the reductive cleavage in stage b).

7. Process according to Claims 1 to 6, characterized in that the reductive cleavage in stage b) is carried out within the temperature range from 35 to 40° C.

8. Process according to Claims 1 to 7, characterized in that the pH is adjusted to a value of 2 to 5 during the reductive cleavage in stage b).

9. Process according to Claims 1 to 8, characterized in that the acetals are cleaved in stage c) by heating with water in the presence of a strongly acid ion exchanger.

10. Process according to Claims 1 to 9, characterized in that acetals of the formula II in which R and $R_1$ denote methyl are employed for the reaction.

**Revendications**

1. Procédé de préparation de glyoxal, d'alcoylglyoxal et de leurs acétals répondant à la formule générale

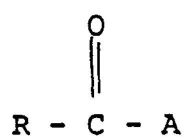 I,

dans laquelle

R représente l'hydrogène ou un reste alcoyle en $C_1$ à $C_6$ linéaire ou ramifié et

A représente le reste -CH=O ou 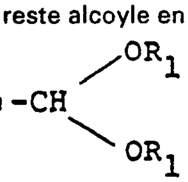 , $R_1$

représentant un reste alcoyle en $C_1$ à $C_6$ linéaire ou ramifié, par ozonolyse d'un dialcoylacétal à insaturations α, β, suivie par l'hydrogénation catalytique du produit d'ozonisation, caractérisé en ce que

a) on dissout un dialcoylglyoxal de l'acroléine ou d'une α-alcoylacroléine répondant à la formule générale

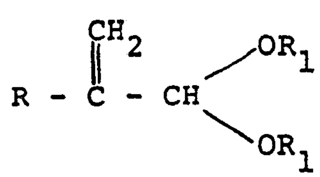 II,

dans laquelle R et $R_1$ ont la signification indiquée dans la formule I, dans un solvant organique et on le fait réagir sur la quantité équivalente d'ozone à des températures de -80 à 0° C,

b) on introduit ensuite en continu la solution obtenue lors de l'ozonisation dans une suspension du catalyseur d'hydrogénation dans le solvant utilisé au stade a) à un dosage tel qu'au cours de toute la durée de l'hydrogénation, il s'établisse ou se main tienne dans la solution d'hydrogénation une teneur en peroxyde de 0,1 mole/litre au maximum, et l'on dissocie par voie réductrice les produits d'ozonisation à une valeur de pH de

2 à 7 et des températures de 15 à 45° C en introduisant de l'hydrogène sous une pression de 1 à 20 bars, à la suite de quoi,

c) si on le souhaite, on dissocie par hydrolyse les acétals obtenus répondant à la formule I où A représente

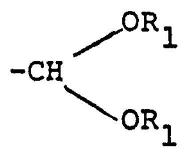

par chauffage avec de l'eau en présence d'acides ou de bases, en le glyoxal correspondant répondant à la formule U où A représente -CH = O.

2. Procédé suivant la revendication 1, caractérisé en ce qu'on effectue l'ozonisation au stade a) à des températures comprises entre -15 et -5° C.

3. Procédé suivant les revendications 1 et 2, caractérisé en ce qu'on utilise, lors de l'ozonisation au stade a) et de la dissociation par réduction des produits d'ozonisation au stade b), un alcool aliphatique inférieur comme solvant.

4. Procédé suivant la revendication 3, caractérisé en ce qu'on utilise comme solvant du méthanol au cours de l'ozonisation du stade a) et de la dissociation par réduction des produits d'ozonisation au stade b).

5. Procédé suivant les revendications 1 à 4, caractérisé en ce que, pour la dissociation par réduction des produits d'ozonisation au stade b), on établit et/ou on maintient une teneur en peroxydes de 0,02 mole/litre au plus dans la solution d'hydrogénation.

6. Procédé suivant les revendications 1 à 5, caractérisé en ce que, pour la dissociation par réduction au stade b), on utilise comme catalyseur du platine sans support.

7. Procédé suivant les revendications 1 à 6, caractérisé en ce qu'on effectue la dissociation par réduction au stade b) dans l'intervalle de température de 35 à 40° C.

8. Procédé suivant les revendications 1 à 7, caractérisé en ce qu'on établit pendant la dissociation par réduction au stade b) une valeur de pH de 2 à 5.

9. Procédé suivant les revendications 1 à 8, caractérisé en ce qu'on dissocie les acétals au stade c) par chauffage avec de l'eau en présence d'un échangeur d'ions fortement acide.

10. Procédé suivant les revendications 1 à 9, caractérisé en ce que, pour la réaction, on utilise des acétals de la formule II où R et $R_1$ représentent un groupe méthyle.